# EUROPEAN PATENT SPECIFICATION

(11) **EP 1 029 863 B1**
(45) Date of publication and mention of the grant of the patent: **14.05.2003**
(21) Application number: 98951712.3
(22) Date of filing: 06.11.1998
(51) Int. Cl.: C07D 471/14, A61K 31/47, A61P 35/00

(54) **CAMPTOTHECIN DERIVATIVES**
CAMPTOTHECIN-DERIVATE
DERIVES DE LA CAMPTOTHECINE

(30) Priority: 06.11.1997 JP 31918297
(43) Date of publication of application: 23.08.2000
(73) Proprietor: Kabushiki Kaisha Yakult Honsha, Minato-ku, Tokyo 105-0021 (JP)
(72) Inventor: OGAWA, Takanori Kabushiki Kaisha Yakult Honsha, Minato-ku Tokyo 105-0021 (JP); YAEGASHI, Takashi Kabushiki Kaisha Yakult Honsha, Minato-ku Tokyo 105-0021 (JP); SAWADA, Seigo Kabushiki Kaisha Yakult Honsha, Minato-ku Tokyo 105-0021 (JP); FURUTA, Tomio Kabushiki Kaisha Yakult Honsha, Minato-ku Tokyo 105-0021 (JP)
(74) Representative: Vossius, Volker, Dr.
(86) International application number: PCT/JP98/05000
(87) International publication number: WO 99/024430

(56) References cited:
- JP-A- 8 073 461
- US-A- 4 914 205

## Description

The present invention relates to new camptothecin derivatives which are water-soluble and excellent in anti-tumor activity.

The present inventors have made an exploratory research on new camptothecin (hereinafter referred to as CPT) derivatives with excellent anti-tumor activity and have already provided many CPT derivatives. Furthermore, it has been found out that in particular a group of derivatives prepared by total synthetis , which have a lower alkyl group in 7-position on the B-ring and various hetero substituents and an alkyl group in the 9-, 10- and 11-position on the A-ring (see JP, A, H1-186892, US, A, 5,061,800) have excellent activity. The present inventors have also made a development research for a method to make said derivatives water-soluble by various means in order to solve an administration problem. It was found that regarding in particular 7-ethyl CPT, the derivatives, wherein the E-lactone ring is opened by use of the diamine and the hydroxymethyl group is protected by the appropriate acyl group, are excellent in water solubility without decrease of anti-tumor activity, contrasting to the E-ring opened water-soluble CPT derivatives known previously (see JP, A, H1-131179, U.S.Pat. No. 4,914,205).

Further, a new group of water-soluble derivatives being excellent in anti-tumor activity was prepared and provided by transforming a group of derivatives having a lower alkyl group in 7-position on the B-ring and various hetero substituents and an alkyl group in the 9-, 10- and 11-position on the A-ring into derivatives wherein the E-lactone ring is opened by the diamine and further the hydroxymethyl group is protected by an appropriate acyl group (see JP, A, H8-73461, AU, Pat. No. 688547). However, though these derivatives solve the water solubility problem, they are unstable in a basic aqueous solution and not necessarily suitable for making a preparation such as a formulation for oral administration .

While realizing that water solubility is favorable for administration, there is need for a used for new CPT derivativer being excellent in anti-tumor activity and in the stability in a basic aqueous solution.

With the object to obtain derivatives having an extremely excellent anti-tumor activity, the present inventors have developed a group of new water-soluble CPT derivatives wherein the stability in a basic aqueous solution is improved by transforming a group of derivatives having a lower alkyl group in 7-position on the B-ring and various hetero substituents and an alkyl group in the 9-, 10-and 11-position on the A-ring into derivatives wherein the E-lactone ring is opened by the diamine and further the hydroxymethyl group is protected by a carbamate type or carbonate type protecting group.

The present invention provides camptothecin derivatives of the general formula (1) 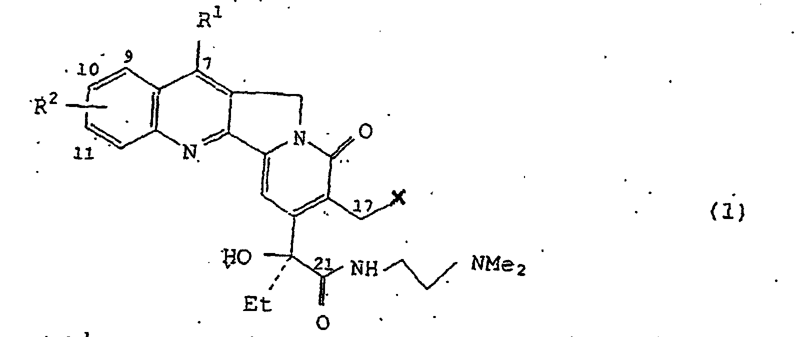 wherein R¹ represents a hydrogen atom or an alkyl group with 1-6 carbon atoms, R² represents the same or different 0-4 alkyl groups with 1-6 carbon atoms, a halogen atom, an alkoxy or hydroxyl group,
wherein X is a carbonate or a carbamate group of the formula 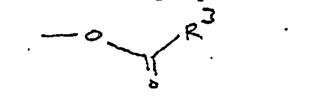 wherein R³ is selected from the group consisting of methylamino, ethylcmino, propylcmino, cyclotexylamino, dimethylamino, diethylamino , arylamino, cyclic-amino, methoxy, ethoxy or propoxy group, and salts thereof.

In the following, the present invention is described in more detail.

### Best Mode for carrying out the Invention

New CPT derivatives according to the present invention can be prepared using as starting material a derivative having a hydrogen atom or an alkyl group having 1-6 carbon atoms in 7-position and various hetero substituents and an alkyl group in the positions 9-12, treating said starting material with N,N-dimethylethylenediamine and subsequently acylating the 17-hydroxyl group with an appropriate acylating agent to give a carbonate or carbamate . Each group of derivatives which may be used as starting material are the known CPT derivatives of natural origin (9-methoxy-CPT, 10-hydroxy-CPT, 10-methoxy-CPT, 11-hydroxy-CPT, 11-methoxy-CPT, etc.) or those obtained by a known semi-synthesis or total synthesis (see the specification of JP, A, 58-39684, JP, A, 58-134095, US, A, 4,473,692, US, A, 4,545,880, JP, A, 59-51287, JP, A, 59-51289, JP, A, H1-279891, US, A, 4,939,255, US, A, 5,061,795, JP, A, H1-186892, US, A, 5,061,800, JP, A, H4-503505, US, A, 4,981,968, JP, A, H5-502017, US, A, 5,049,668, WO91/04260, WO92/11263, US, A, 5,122,606, etc.).

Specifically, R¹ in the general formula can be selected from a hydrogen atom of the natural type, or an alkyl group with 1-6 carbon atoms, wherein methyl, ethyl or propyl groups are particularly preferred.

R² in the general formula represents 0-4 substituents on the A-ring (9- to 12-position), though specifically, in the 9-, 10-, 11- or 12-positions can be selected those substituted independently or together by hydroxyl, a lower alkoxy group such as methoxy, a halogen such as fluorine, chlorine or bromine, or an alkyl group with 1-6 carbon atoms such as methyl or ethyl, wherein compounds having a fluoro group in the 11-position and compounds having a methyl group in the 10-position are preferred, in particular compounds having a fluoro group in the 11-position and a methyl group in the 10-position together are preferred.

Further, R³ in the general formula represents a methylamino, ethylamino, propylamino or cyclohexylamino group, a dimethylamino or diethylamino group, an arylamino group such as a phenylamino or fluorophenylamino group, a cyclic-amino group such as a 4-isopropylaminocarbonylpiperazinyl group, or a methoxy, ethoxy or propoxy group. Among these phenylamino, dimethylamino and methoxy groups are particularly preferred.

The treatment with N,N-dimethylethylenediamine and the subsequent acylation of the 17-hydroxyl group by an acylating agent can be carried out according to the method disclosed by the present inventors in JP, A, H1-131179.

In particular, the opening of the E-lactone ring by N,N-dimethylethylenediamine is carried out in the absence of a reaction solvent and by using only an excess amount of N,N-dimethylethylenediamine permitting the conversion into a E-ring opened intermediate. Subsequently, the desired substance can be obtained in a high yield by acylating the 17-hydroxyl group with an appropriate acylating agent.

The acylating agents which can be used in the above acylation reaction are not specifically limited. Illustrative of these are the corresponding alkylisocyanates, arylisocyanates, or the corresponding alkylcarbamoylchlorides or chloroalkylcarbonates.

In the acylation reaction, pyridine, N,N-dimethylaminopyridine or the like may be present together in the reaction mixture as a catalyst. Further, it is possible to improve the yield of a desired substance by keeping the reaction conditions as anhydrous as possible except the ring opening reaction, that is, the acylation process, or in the later work-up processes such as a pulverization, purification and crystallization processes. The new CPT derivatives according to the invention show excellent water- solubility as salts with an appropriate acid such as hydrochloric acid, and excellent stability in basic aqueous solution. Further, the test results as regards their anti-tumor effects reveal that they have excellent properties and are extremely useful as new anti-tumor agents.

In the following, the present invention will be explained in more detail by way of examples.

### Example 1: 7-Ethyl-17-methylcarbamoyloxycamptothecin-21-(2-dimethylamino) ethylamide (compound 1)

To a solution of 1.00 g (2.16 mmol) 7-ethyl-17-hydroxycamptothecin-21-(2-dimethylamino) ethylamide in 5 ml methylene chloride (dried on MS4Å) were added 0.11 ml (1.81 mmol, 1.2 ep.) methyl isocyanate in an atmosphere of argon under ice-cooling and stirring. After stirring for half an hour, the mixture was stirred at room temperature (25°C) for 6 hr. The reaction solution was purified directly by medium pressure silica gel chromatography (CHCl₃:MeOH) to give 582 mg (52%) of the title compound as pale yellow crystals.
mp 188-190°C (decomp)
IR (KBr) 3300, 1685, 1645, 1590, 1510, 1260 cm⁻¹
¹H-NMR (400 MHz, CDCl₃) δ 1.07 (t, J=7.3 Hz, 3H, 18-CH₃), 1.34 (t, J=7.7 Hz, 3H, 7-CH₂CH₃), 2.23-2.52 (m, 4H, CH₂N(CH₃)₂ and 19-CH₂), 2.26 (s, 6H, N(CH₃)₂), 2.76 and 2.78 (s, 3H, NHCH₃ two rotamors), 3.06-3.11 (m, 2H, 7-CH₂CH₃), 3.34-3.45 (m, 2H, CONHCH₂), 5.05 (d, J=18.8 Hz, 1H, 5-CH₂), 5.12 (d, J=18.8 Hz, 1H, 5-CH₂), 5.35 (d, J=12.0 Hz, 1H, 17-OCH₂), 5.61 (d, J=12.0 Hz, 1H, 17-OCH₂), 7.48-7.50 (m, 1H, 10-H), 7.59 (s, 1H, 14-H), 7.67-7.69 (m, 1H, 11-H), 7.88 (d, J=8.3 Hz, 1H, 9-H), 8.08 (d, J=8.3 Hz, 1H, 12-H)
SI-MS m/e 522 (M⁺+1)

### Example 2: 7-Ethyl-17-ethylcarbamoyloxycamptothecin-21-(2-dimethylamino)ethylamide (compound 2)

Except using ethyl isocyanate, the reaction and the after treatment were carried out according to the method of example 1 to give 212 mg (18%) of the title compound as pale yellow crystals.
mp 168-170°C (decomp)
IR (KBr) 3305, 1685, 1650, 1595, 1510, 1255 cm⁻¹
¹H-NMR (400 MHz, CDCl₃) δ 1.06 (t, J=7.2 Hz, 3H, 18-CH₃), 1.12 (t, J=7.2 Hz, 3H, NHCH₂CH₃), 1.34 (t, J=7.7 Hz, 3H, 7-CH₂CH₃), 2.25-2.54 (m, 4H, CH₂N(CH₃)₂ and 19-CH₂), 2.29 (s, 6H, N(CH₃)₂), 3.02-3.11 (m, 2H, 7-CH₂CH₃), 3.16-3.23 (m, 2H, NHCH₂CH₃), 3.32-3.51 (m, 2H, CONHCH₂), 5.01 (d, J=18.7 Hz, 1H, 5-CH₂), 5.08 (d, J=18.7 Hz, 1H, 5-CH₂), 5.34 (d, J=11.8 Hz, 1H, 17-OCH₂), 5.57 (d, J=11.8 Hz, 1H, 17-OCH₂), 7.40-7.48 (m, 1H, 10-H), 7.58 (s, 1H, 14-H), 7.61-7.69 (m, 1H, 11-H), 7.83 (d, J=8.3 Hz, 1H, 9-H), 8.03 (d, J=8.3 Hz, 1H, 12-H)
SI-MS m/e 536 (M⁺+1)

### Example 3: 7-Ethyl-17-n-propylcarbamoyloxycamptothecin-21-(2-dimethylamino)ethylamide (compound 3)

Except using n-propyl isocyanate, the reaction and the after treatment were carried out according to the method of example 1 to give 240 mg (20%) of the title compound as pale yellow crystals.
mp 170-171°C (decomp)
IR (KBr) 3320, 1680, 1650, 1590, 1510, 1265 cm⁻¹
¹H-NMR (400 MHz, CDCl₃) δ 0.89 (t, J=7.3 Hz, 3H, CH₂CH₂CH₃), 1.08 (t, J=7.2 Hz, 3H, 18-CH₃), 1.34 (t, J=7.7 Hz, 3H, 7-CH₂CH₃), 1.47-1.53 (m, 2H, CH₂CH₂CH₃), 2.25 (s, 6H, N(CH₃)₂), 2.25-2.50 (m, 4H, CH₂N(CH₃)₂ and 19-CH₂), 3.07-3.14 (m, 4H, 7-CH₂CH₃ and NHCH₂CH₂CH₃), 3.36-3.47 (m, 2H, CONHCH₂), 5.05 (d, J=18.8 Hz, 1H, 5-CH₂), 5.11 (d, J=18.8 Hz, 1H, 5-CH₂), 5.34 (d, J=12.1 Hz, 1H, 17-OCH₂), 5.65 (d, J=12.1 Hz, 1H, 17-OCH₂), 6.83 (brs, 1H, OH), 7.47-7.51 (m, 1H, 10-H), 7.56-7.61 (m, 1H, NH), 7.60 (s, 1H, 14-H), 7.67-7.70 (m, 1H, 11-H), 7.88 (d, J=8.3 Hz, 1H, 9-H), 8.08 (d,
J=8.5 Hz, 1H, 12-H)
SI-MS m/e 550(M⁺+1)

### Example 4: 7-Ethyl-17-iso-propylcarbamoyloxycamptothecin-21-(2-dimethylamino)ethylamide (compound 4)

Except using iso-propyl isocyanate, the reaction and the after treatment were carried out according to the method of example 1 to give 938 mg (79%) of the title compound as pale yellow crystals.
mp 179-180°C (decomp)
IR (KBr) 3300, 1685, 1650, 1595, 1510, 1250 cm⁻¹
¹H-NMR (400 MHz, CDCl₃) δ 1.06 (t, J=7.2 Hz, 3H, 18-CH₃), 1.11 (d, J=6.6 Hz, 3H, CH(CH₃)₂), 1.14 (d, J=6.6 Hz, 3H, CH(CH₃)₂), 1.36 (t, J=7.6 Hz, 3H, 7-CH₂CH₃), 2.29 (s, 6H, N(CH₃)₂), 2.31-2.51 (m, 4H, CH₂N(CH₃)₂ and 19-CH₂), 3.09-3.15 (m, 2H, 7-CH₂CH₃), 3.34-3.48 (m, 2H, CONHCH₂), 3.73-3.82 (m, 1H, CH(CH₃)₂), 5.09 (d, J=18.8 Hz, 1H, 5-CH₂), 5.16 (d, J=18.8 Hz, 1H, 5-CH₂), 5.31 (d, J=11.6 Hz, 1H, 17-OCH₂), 5.69 (d, J=11.6 Hz, 1H, 17-OCH₂), 7.53-7.57 (m, 2H, 10-H and NH), 7.63 (s, 1H, 14-H), 7.69-7.73 (m, 1H, 11-H), 7.94-7.96 (m, 1H, 9-H), 8.11-8.13 (m, 1H, 12-H)
SI-MS m/e 550(M⁺+1)

### Example 5: 17-n-Butylcarbamoyloxy-7-ethylcamptothecin-21-(2-dimethylamino)ethylamide (compound 5)

Except using n-butyl isocyanate, the reaction and the after treatment were carried out according to the method of example 1 to give 598 mg (49%) of the title compound as pale yellow crystals.
mp 170-172°C (decomp)
IR (KBr) 3300, 1680, 1650, 1590, 1510, 1460, 1250 cm⁻¹ ¹H-NMR (400 MHz, CDCl₃) δ 0.89 (t, J=7.3 Hz, 3H, NCH₂CH₂CH₂CH₃), 1.05 (t, J=7.2 Hz, 3H, 18-CH₃), 1.26-1.49 (m, 4H, CH₂CH₂CH₂CH₃), 1.36 (t, J=7.7 Hz, 3H, 7-CH₂CH₃), 2.28-2.53 (m, 4H, CH₂N(CH₃)₂ and 19-CH₂), 2.31 (s, 6H, N(CH₃)₂), 3.10-3.17 (m, 4H, 7-CH₂CH₃ and NHCH₂CH₂CH₂CH₃), 3.35-3.46 (m, 2H, CONHCH₂), 5.11 (d, J=18.5 Hz, 2H, 5-CH₂), 5.14 (d, J=18.5 Hz, 2H, 5-CH₂), 5.23 (brs, 1H, NH), 5.33 (d, J=12.1 Hz, 1H, 17-OCH₂), 5.69 (d, J=12.1 Hz, 1H, 17-OCH₂), 6.46 (brs, 1H, OH), 7.55-7.59 (m, 2H, 10-H and NH), 7.64 (s, 1H, 14-H), 7.70-7.74 (m, 1H, 11-H), 7.97 (d, J=7.6 Hz, 1H, 9-H), 8.13 (d, J=8.3 Hz, 1H, 12-H)
SI-MS m/e 564(M⁺+1)

### Example 6: 7-Ethyl-17-cyclo-hexylcarbamoyloxycamptothecin-21-(2-dimethylamino)ethylamide (compound 6)

Except using cyclohexyl isocyanate, the reaction and the after treatment were carried out according to the method of example 1 to give 888 mg (70%) of the title compound as pale yellow crystals.
mp 176-179°C (decomp)
IR (KBr) 3290, 1650, 1595, 1510, 1450, 1230 cm⁻¹
¹H-NMR (400 MHz, CDCl₃) δ 1.05-1.37 (m, 3H, CH₂), 1.06 (t, J=7.2 Hz, 3H, 18-CH₃), 1.36 (t, J=7.6 Hz, 3H, 7-CH₂CH₃), 1.52-1.72 (m, 3H, CH₂), 1.82-1.93 (m, 2H, CH₂), 2.05-2.21 (m, 2H, CH₂), 2.29 (s, 6H, N(CH₃)₂), 2.32-2.52 (m, 4H, CH₂N(CH₃)₂ and 19-CH₂), 3.09-3.15 (m, 2H, 7-CH₂CH₃), 3.34-3.49 (m, 3H, CONHCH₂ and NHCH(CH₂)₂), 5.09 (d, J=18.5 Hz, 1H, 5-CH₂), 5.16 (d, J=18.5 Hz, 1H, 5-CH₂), 5.30 (d, J=12.0 Hz, 1H, 17-OCH₂), 5.69 (d, J=12.0 Hz, 1H, 17-OCH₂), 7.52-7.60 (m, 2H, 10-H and NH), 7.64 (s, 1H, 14-H), 7.69-7.71 (m, 1H, 11-H), 7.95 (d, J=8.3 Hz, 1H, 12-H), 8.13 (d, J=8.3 Hz, 1H, 12-H)
SI-MS m/e 590(M⁺+1)

### Example 7: 7-Ethyl-17-phenylcarbamoyloxycamptothecin-21-(2-dimethylamino)ethylamide (compound 7)

Except using phenyl isocyanate, the reaction and the after treatment were carried out according to the method of example 1 to give 650 mg (52%) of the title compound as pale yellow crystals.
mp 140-146°C (decomp)
IR (KBr) 3315, 1715, 1680, 1650, 1595, 1530, 1210 cm⁻¹
¹H-NMR (400 MHz, CDCl₃) δ 1.07 (t, J=7.1 Hz, 3H, 18-CH₃), 1.30 (t, J=7.6 Hz, 3H, 7-CH₂CH₃), 2.13 (s, 6H, N(CH₃)₂), 2.13-2.53 (m, 4H, CH₂N(CH₃)₂ and 19-CH₂), 2.89-3.05 (m, 2H, 7-CH₂CH₃), 3.30-3.45 (m, 2H, CONHCH₂), 4.90 (d, J=18.7 Hz, 1H, 5-CH₂), 4.98 (d, J=18.7 Hz, 1H, 5-CH₂), 5.51 (d, J=11.5 Hz, 1H, 17-OCH₂), 5.54 (d, J=11.5 Hz, 1H, 17-OCH₂), 5.85 (brs, 1H, OH), 6.94-6.98 (m, 1H, C₆H₅-p), 7.19-7.23 (m, 2H, C₆H₅-m), 7.33-7.37 (m, 1H, 10-H), 7.42-7.44 (m, 2H, C₆H₅-o), 7.50 (s, 1H, 14-H), 7.56-7.60 (m, 1H, 11-H), 7.67 (d, J=8.4 Hz, 1H, 9-H), 7.92 (d, J=8.3 Hz, 1H, 12-H).
SI-MS m/e 584(M⁺+1)

### Example 8: 7-Ethyl-17-(2-fluorophenylcarbamoyloxy)-camptothecin-21-(2-dimethylamino)ethylamide (compound 8)

Except using 2-fluorophenyl isocyanate, the reaction and the after treatment were carried out according to the method of example 1 to give 793 mg (61%) of the title compound as pale yellow crystals.
mp 120-125°C (decomp)
IR (KBr) 3385, 1715, 1650, 1595, 1520, 1220 cm⁻¹
¹H-NMR (400 MHz, CDCl₃) δ 1.08 (t, J=7.2 Hz, 3H, 18-CH₃), 1.35 (t, J=7.7 Hz, 3H, 7-CH₂CH₃), 2.27 (s, 6H, N(CH₃)₂), 2.31-2.57 (m, 4H, CH₂N(CH₃)₂ and 19-CH₂), 3.05-3.12 (m, 2H, 7-CH₂CH₃), 3.34-3.52 (m, 2H, CONHCH₂), 5.08 (d, J=18.8 Hz, 1H, 5-CH₂), 5.15 (d, J=18.8 Hz, 1H, 5-CH₂), 5.56 (d, J=11.7 Hz, 1H, 17-OCH₂), 5.75 (d, J=11.7 Hz, 1H, 17-OCH₂), 6.96-7.08 (m, 3H, C₆H₄-F), 7.31 (brs, 1H, NH), 7.50-7.54 (m, 2H, 10-H and C₆H₄-F), 7.62 (s, 1H, 14-H), 7.68-7.73 (m, 1H, 11-H), 7.90 (d, J=8.8 Hz, 1H, 9-H), 8.06-8.10 (m, 2H, 12-H and NH)
SI-MS m/e 602(M⁺+1)

### Example 9: 7-Ethyl-17-(3-fluorophenylcarbamoyloxy)-camptothecin-21-(2-dimethylamino)ethylamide (compound 9)

Except using 3-fluorophenyl isocyanate, the reaction and the after treatment were carried out according to the method of example 1 to give 1.20 g (92%) of the title compound as pale yellow crystals.
mp 143-147°C (decomp)
IR (KBr) 3275, 1715, 1648, 1594, 1542, 1218 cm⁻¹
¹H-NMR (400 MHz, CDCl₃) δ 1.06 (t, J=7.2 Hz, 3H, 18-CH₃), 1.28 (t, J=7.6 Hz, 3H, 7-CH₂CH₃), 2.11-2.54 (m, 4H, CH₂N(CH₃)₂ and 19-CH₂), 2.14 (s, 6H, N(CH₃)₂), 2.85-2.98 (m, 2H, 7-CH₂CH₃), 3.23-3.28 (m, 1H, CONHCH₂), 3.39-3.44 (m, 1H, CONHCH₂), 4.82 (d, J=18.8 Hz, 1H, 5-CH₂), 4.90 (d, J=18.8 Hz, 1H, 5-CH₂), 5.43 (s, 2H, 17-OCH₂), 6.47-6.49 (m, 1H, NH), 6.99-7.06 (m, 2H, C₆R₄-F), 7.28-7.32 (m, 2H, C₆H₄-F and 10-H), 7.45 (s, 1H, 14-H), 7.52-7.64 (m, 3H, 11-H and 9-H and C₆H₄-F), 7.85 (d, J=8.3 Hz, 1H, 12-H), 8.32 (brs, 1H, NH)
SI-MS m/e 602(M⁺+1)

### Example 10: 7-Ethyl-17-(4-fluorophenylcarbamoyloxy)-camptothecin-21- (2-dimethylamino) ethylamide (compound 10)

Except using 4-fluorophenyl isocyanate, the reaction and the after treatment were carried out according to the method of example 1 to give 1.20 g (93%) of the title compound as pale yellow crystals.
mp 138-143°C (decomp)
IR (KBr) 3275, 1715, 1650, 1595, 1540, 1220 cm⁻¹
¹H-NMR (400 MHz, CDCl₃) δ 1.06 (t, J=7.2 Hz, 3H, 18-CH₃), 1.32 (t, J=7.7 Hz, 3H, 7-CH₂CH₃), 2.17 (s, 6H, N(CH₃)₂), 2.24-2.52 (m, 4H, CH₂N(CH₃)₂ and 19-CH₂), 2.99-3.05 (m, 2H, 7-CH₂CH₃), 3.29-3.45 (m, 2H, CONHCH₂), 4.94 (d, J=18.5 Hz, 1H, 5-CH₂), 5.02 (d, J=18.5 Hz, 1H, 5-CH₂), 5.46 (d, J=11.6 Hz, 1H, 17-OCH₂), 5.54 (d, J=11.6 Hz, 1H, 17-OCH₂), 6.87-6.91 (m, 2H, C₆H₄-m-F), 7.33-7.37 (m, 2H, C₆H₄-o-F), 7.38-7.42 (m, 1H, 10-H), 7.50 (s, 1H, 14-H), 7.59-7.62 (m, 2H, 11-H and NH), 7.74 (d, J=8.3 Hz, 1H, 9-H), 7.83 (brs, 1H, NH), 7.94 (d, J=8.5 Hz, 1H, 12-H)
SI-MS m/e 602(M⁺+1)

### Example 11: 7-Ethyl-17-(1-naphthylcarbamoyloxy)-camptothecin-21-(2-dimethylamino)ethylamide (compound 11)

Except using 1-naphthyl isocyanate, the reaction and the after treatment were carried out according to the method of example 1 to give 1.08 g (79%) of the title compound as pale yellow crystals.
mp 155-159°C (decomp)
IR (KBr) 3350, 1690, 1650, 1595, 1520, 1455, 1215 cm⁻¹
¹H-NMR (400 MHz, DMSO-d₆) δ 0.93 (t, J=7.2 Hz, 3H, 18-CH₃), 1.35 (t, J=7.6 Hz, 3H, 7-CH₂CH₃), 2.09 (s, 6H, N(CH₃)₂), 2.12-2.34 (m, 4H, CH₂N(CH₃)₂ and 19-CH₂), 3.16-3.29 (m, 4H, 7-CH₂CH₃ and CONHCH₂), 5.34 (s, 2H, 5-CH₂), 5.43 (d, J=10.7 Hz, 1H, 17-OCH₂), 5.57 (d, J=10.7 Hz, 1H, 17-OCH₂), 6.33 (s, 1H, OH), 7.47-7.52 (m, 2H), 7.49 (s, 1H, 14-H), 7.63-7.65 (m, 1H), 7.71-7.76 (m, 2H), 7.84-7.92 (m, 3H), 8.08-8.11 (m, 1H), 8.20 (d, J=8.5 Hz, 1H, 9-H), 8.30 (d, J=9.0 Hz, 1H, 12-H)
SI-MS m/e 634(M⁺+1)

### Example 12: 7-Ethyl-10-methyl-17-phenylcarbamoyloxycamptothecin-21-(2-dimethylamino)ethylamide (compound 12)

Except using 7-Ethyl-10-methyl-17-hydroxycamptothecin-21-(2-dimethylamino)ethylamide and phenyl isocyanate, the reaction and the after treatment were carried out according to the method of example 1 to give 872 mg (70%) of the title compound as pale yellow crystals.
mp 155-157°C (decomp)
IR (KBr) 3285, 1700, 1650, 1595, 1525, 1200 cm⁻¹
¹H-NMR (400 MHz, CDCl₃) δ 1.05 (t, J=7.1 Hz, 3H, 18-CH₃), 1.30 (t, J=7.6 Hz, 3H, 7-CH₂CH₃), 2.16 (s, 6H, N(CH₃)₂), 2.20-2.54 (m, 4H, CH₂N(CH₃)₂ and 19-CH₂), 2.48 (s, 3H, 10-CH₃), 2.89-3.07 (m, 2H, 7-CH₂CH₃), 3.23-3.47 (m, 2H, CONHCH₂), 4.94 (d, J=18.7 Hz, 1H, 5-CH₂), 5.03 (d, J=8.7 Hz, 1H, 5-CH2), 5.49 (d, J=11.7 Hz, 1H, 17-OCH₂), 5.57 (d, J=11.7 Hz, 1H, 17-OCH₂), 5.73 (brs, 1H, OH), 6.96-7.02 (m, 1H, C₆H₅-p), 7.19-7.27 (m, 2H, C₆H₅-m), 7.38-7.56 (m, 7H, C₆H₅-o, 14-H, 11-H, 9-H, NH), 7.83 (d, J=8.8 Hz, 1H, 12-H)
SI-MS m/e 598(M⁺+1)

### Example 13: 7-Ethyl-11-fluoro-17-phenylcarbamoyloxycamptothecin-21-(2-dimethylamino)ethylamide (compound 13)

Except using 7-ethyl-11-fluoro-17-hydroxy-camptothecin-21-(2-dimethylamino)ethylamide and phenyl isocyanate, the reaction and the after treatment were carried out according to the method of example 1 to give of 1.22 g (98%) the title compound as pale yellow crystals.
mp 155-157°C (decomp)
IR (KBr) 3280, 1720, 1695, 1650, 1595, 1510, 1440, 1310, 1210 cm⁻¹
¹H-NMR (400 MHz, CDCl₃) δ 1.08 (t, J=7.3 Hz, 3H, 18-CH₃), 1.36 (t, J=7.6 Hz, 3H, 7-CH₂CH₃), 2.17 (s, 6H, N(CH₃)₂), 2.24-2.55 (m, 4H, CH₂N(CH₃)₂ and 19-CH₂), 3.01-3.12 (m, 2H, 7-CH₂CH₃), 3.27-3.47 (m, 2H, CONHCH₂), 4.96 (d, J=18.6 Hz, 1H, 5-CH₂), 5.04 (d, J=18.6 Hz, 1H, 5-CH₂), 5.49 (d, J=11.8 Hz, 1H, 17-OCH₂), 5.58 (d, J=11.8 Hz, 1H, 17-OCH₂), 5.78 (brs, 1H, OH), 6.97-7.01 (m, 1H, C₆H₅-p), 7.17-7.25 (m, 1H, 10-H), 7.21-7.25 (m, 2H, C₆H₅-m), 7.42 (d, J=7.8 Hz, 2H, C₆H₅-o), 7.49 (s, 1H, 14-H), 7.51 (dd, J=2.4, 10.0 Hz, 1H, 12-H), 7.58 (brt, J=5.2 Hz, 1H, CONH), 7.81 (dd,
J=6.0, 9.4 Hz, 1H, 9-H)
SI-MS m/e 602(M⁺+1)

### Example 14: 17-Dimethylcarbamoyloxy-7-ethylcamptothecin-21-(2-dimethylamino)ethylamide (compound 14)

To a solution of 500 mg (1.08 mmol) 7-ethyl-17-hydroxycamptothecin-21-(2-dimethylamino)ethylamide and 0.25 ml (3.1 mmol, 2.9 %) pyridine in 5ml methylene chloride ( dried on MS4Å) was added 0.15 ml (1.62 mmol, 1.5 eq.) dimethylcarbamoyl chloride in an atmosphere of argon under ice-cooling and stirring. After stirring for half an hour, the mixture was stirred at room temperature (25°C) for 16 hr. To the reaction solution were added 2ml of a saturated aqueous sodium bicarbonate solution and 0.1 hr later 50 ml chloroform , and the organic phase was washed with 10 ml water, 10 ml of a saturated aqueous sodium chloride solution , and dried over anhydrous sodium sulfate. The solvent was removed under reduced pressure, and the resulting yellow oil (710 mg) was purified by silica gel chromatography (CHCl₃:MeOH) to give 273 mg (47%) of the title compound as pale yellow crystals.
mp 195-200°C (decomp)
IR (KBr) 3380, 1675, 1650, 1595, 1510, 1455, 1190 cm⁻¹
¹H-NMR (400 MHz, CDCl₃) δ 1.00 (t, J=7.3 Hz, 3H, 18-CH₃), 1.37 (t, J=7.7 Hz, 3H, 7-CH₂CH₃), 2.39 (q, J=7.3 Hz, 2H, 19-CH₂), 2.65 (s, 6H, CH₂N(CH₃)₂), 2.90 (s, 6H, CON(CH₃)₂), 2.92-3.01 (m, 2H, CH₂N(CH₃)₂), 3.15 (q, J=7.7 Hz, 2H, 7-CH₂CH₃), 3.46-3.52 (m, 1H, CONHCH₂), 3.73-3.78 (m, 1H, CONHCH₂), 5.16 (d, J=18.9 Hz, 1H, 5-CH₂), 5.22 (d, J=18.9 Hz, 1H, 5-CH₂), 5.51 (d, J=11.8 Hz, 1H, 17-OCH₂), 5.62 (d, J=11.8 Hz, 1H, 17-OCH₂), 6.67 (brs, 1H, OH), 7.60-7.63 (m, 1H, 10-H), 7.63 (s, 1H, 14-H), 7.74-7.78 (m, 1H, 11-H), 7.94-7.95 (m, 1H, NH), 8.06 (d, J=8.3 Hz, 1H, 9-H), 8.18 (d, J=8.3 Hz, 1H, 12-H)
SI-MS m/e 536(M⁺+1)

### Example 15: 17-Diethylcarbamoyloxy-7-ethylcamptothecin-21-(2-dimethylamino)ethylamide (compound 15)

Except using diethylcarbamoyl chloride, the reaction and the after treatment were carried out according to the method of example 14 to give 132 mg (22%) of the title compound as pale yellow crystals.
mp 117-122°C (decomp)
IR (KBr) 3380, 1650, 1595, 1510, 1475, 1275 cm⁻¹
¹H-NMR (400 MHz, CDCl₃) δ 0.97 (t, J=7.3 Hz, 3H, 18-CH₃), 1.11 (t, J=7.1 Hz, 6H, CON(CH₂CH₃)₂), 1.37 (t, J=7.7 Hz, 3H, 7-CH₂CH₃), 2.34-2.45 (m, 2H, 19-CH₂), 2.82 (s, 6H, CH₂N(CH₃)₂), 3.12-3.36 (m, 8H, CH₂N(CH₃)₂ and 7-CH₂CH₃ and CON(CH₂CH₃)₂), 3.52-3.57 (m, 1H, CONHCH₂), 3.80-3.91 (m, 1H, CONHCH₂), 5.17 (d, J=18.8 Hz, 1H, 5-CH₂), 5.22 (d, J=18.8 Hz, 1H, 5-CH₂), 5.59 (s, 2H, 17-OCH₂), 6.77 (brs, 1H, OH), 7.61 (s, 1H, 14-H), 7.61-7.65 (m, 1H, 10-H), 7.75-7.78 (m, 1H, 11-H), 8.06 (brs, 1H, NH), 8.07 (d, J=8.1 Hz, 1H, 9-H), 8.18 (d, J=8.1 Hz, 1H, 12-H)
SI-MS m/e 564 (M⁺+1)

### Example 16: 7-Ethyl-17-(4-isopropylaminocarbonylmethylpiperazino)-carbamoyloxy-camptothecin-21-(2-dimethylamino)ethylamide (compound 16)

Except using 4-(isopropylaminocarbonylmethylpiperazino) carbamoyl chloride, the reaction and the after treatment were carried out according to the method of example 14 to give 448 mg (41%) of the title compound as pale yellow crystals.
mp 122-123°C (decomp)
IR (KBr) 3315, 1650, 1595, 1515, 1455, 1420, 1235 cm⁻¹
¹H-NMR (400 MHz, CDCl₃) δ 1.03 (t, J=7.3 Hz, 3H, 18-CH₃), 1.15 (d, J=6.3 Hz, 6H, CH(CH₃)₂), 1.38 (t, J=7.7 Hz, 3H, 7-CH₂CH₃), 2.37-2.75 (m, 8H, CH₂N(CH₃)₂ and 19-CH₂ and CH₂N×2), 2.48 (s, 6H, N(CH₃)₂), 2.96 (s, 2H, NCH₂CO), 3.14-3.20 (m, 2H, 7-CH₂CH₃), 3.42-3.51 (m, 6H, CONHCH₂ and OCONCH₂×2), 4.05-4.11 (m, 1H, CONHCH(CH₃)₂), 5.17 (d, J=18.7 Hz, 1H, 5-CH₂), 5.23 (d, J=18.7 Hz, 1H, 5-CH₂), 5.47 (d, J=11.6 Hz, 1H, 17-OCH₂), 5.69 (d, J=11.6 Hz, 1H, 17-OCH₂), 6.83 (d, J=8.1 Hz, 1H, CONHCH(CH₃)₂), 7.61-7.65 (m, 1H, 10-H), 7.65 (s, 1H, 14-H), 7.76-7.79 (m, 2H, 11-H and NH), 8.08 (d, J=8.3 Hz, 1H, 9-H), 8.20 (d, J=9.0 Hz, 1H, 12-H) SI-MS m/e 676(M⁺+1)

### Example 17: 17-Dimethylcarbamoyloxy-7-ethyl-11-fluoro-10-methylcamptothecin-21-(2-dimethylamino)ethylamide (compound 17)

Except using 7-ethyl-11-fluoro-17-hydroxy-10-methylcamptothecin-21-(2-dimethylamino)ethylamide and dimethylcarbamoyl chloride, the reaction and the after treatment were carried out according to the method of example 14 to give 167 mg (27%) of the title compound as pale yellow crystals.
mp 208-212°C (decomp)
IR (KBr) 3380, 1670, 1605, 1510, 1450, 1190 cm⁻¹
¹H-NMR (400 MHz, CDCl₃) δ 0.98 (t, J=7.3 Hz, 3H, 18-CH₃), 1.34 (t, J=7.8 Hz, 3H, 7-CH₂CH₃), 2.28-2.42 (m, 2H, 19-CH₂), 2.50 (s, 3H, 10-CH₃), 2.56 (s, 6H, CH₂N(CH₃)₂), 2.78-2.98 (m, 2H, CH₂N(CH₃)₂), 2.87 (s, 6H, CON(CH₃)₂), 3.10 (q, J=7.8 Hz, 2H, 7-CH₂CH₃), 3.38-3.50 (m, 1H, CONHCH₂), 3.60-3.74 (m, 1H, CONHCH₂), 5.11 (d, J=18.8 Hz, 1H, 5-CH₂), 5.17 (d, J=18.8 Hz, 1H, 5-CH₂), 5.44 (d, J=11.8 Hz, 1H, 17-OCH₂), 5.61 (d, J=11.8 Hz, 1H, 17-OCH₂), 6.71 (brs, 1H, OH), 7.57 (s, 1H, 14-H), 7.71 (d, J=10.7 Hz, 1H, 12-H), 7.77-7.90 (br, 1H, NH), 7.81 (d, J=7.8 Hz, 1H, 9-H)
SI-MS m/e 568(M⁺+1)

### Example 18: 7-Ethyl-17-methoxycarbonyloxycamptothecin-21-(2-dimethylamino)ethylamide (compound 18)

To a solution of 200 mg (0.431 mmol) 7-ethyl-17-hydroxycamptothecin-21-(2-dimethylamino)ethylamide and 0.1 ml (1.24 mmol, 2.9 eq.) pyridine in 2ml methylene chloride were added 0.04 ml (0.521 mmol, 1.2 eq.) methyl chlorocarbonate in an atmosphere of argon under ice-cooling and stirring. After stirring for half an hour, to the reaction solution were added 2 ml a saturated aqueous sodium bicarbonate solution and the resulting solution was stirred for 0.1 hr. 20 ml chloroform were added to the mixture, and the organic phase was washed with 10 ml water, 10 ml of a saturated aqueous sodium chloride solution , and dried over anhydrous sodium sulfate. The solvent was removed under reduced pressure, and the resulting yellow oil (250 mg) was purified by silica gel chromatography (CHCl₃:MeOH) to give 220 mg (98%) of the title compound as pale yellow crystals.
mp 132-139°C (decomp)
IR (KBr) 3370, 1740, 1645, 1595, 1510, 1440, 1265 cm⁻¹
¹H-NMR (400 MHz, CDCl₃) δ 1.08 (t, J=7.3 Hz, 3H, 18-CH₃), 1.35 (t, J=7.6 Hz, 3H, 7-CH₂CH₃), 2.17-2.52 (m, 4H, CH₂N(CH₃)₂ and 19-CH₂), 2.21 (s, 6H, N(CH₃)₂), 3.05-3.16 (m, 2H, 7-CH₂CH₃), 3.34-3.46 (m, 2H, CONHCH₂), 3.79 (s, 3H, OCH₃), 5.09 (s, 2H, 5-CH₂), 5.61 (s, 2H, 17-OCH₂), 7.38-7.42 (m, 1H, NH), 7.56 (s, 1H, 14-H), 7.56-7.59 (m, 1H, 10-H), 7.72-7.76 (m, 1H, 11-H), 7.97 (d, J=8.1 Hz, 1H, 9-H), 8.12 (d, J=8.3 Hz, 1H, 12-H)
SI-MS m/e 523(M⁺+1)

### Example 19: 7-Ethyl-17-ethoxycarbonyloxycamptothecin-21-(2-dimethylamino)ethylamide (compound 19)

Except using ethyl chlorocarbonate, the reaction and the after treatment were carried out according to the method of example 18 to give 153 mg (66%) of the title compound as pale yellow crystals.
mp 95-98°C (decomp)
IR (KBr) 3365, 1740, 1645, 1595, 1505, 1455, 1255 cm⁻¹
¹H-NMR (400 MHz, CDCl₃) δ 1.06 (t, J=7.3 Hz, 3H, 18-CH₃), 1.29 (t, J=7.1 Hz, 3H, OCH₂CH₃), 1.35 (t, J=7.7 Hz, 3H, 7-CH₂CH₃), 2.20-2.52 (m, 4H, CH₂N(CH₃)₂ and 19-CH₂), 2.25 (s, 6H, N(CH₃)₂), 3.06-3.16 (m, 2H, 7-CH₂CH₃), 3.32-3.53 (m, 2H, CONHCH₂), 4.21 (q, J=7.1 Hz, 2H, OCH₂CH₃), 5.09 (s, 2H, 5-CH₂), 5.60 (s, 2H, 17-OCH₂), 7.38-7.42 (m, 1H, NH), 7.56-7.60 (m, 1H, 10-H), 7.57 (s, 1H, 14-H), 7.72-7.76 (m, 1H, 11-H), 7.98 (d, J=8.1 Hz, 1H, 9-H), 8.13 (d, J=7.8 Hz, 1H, 12-H)
SI-MS m/e 535(M⁺-1)

### Example 20: 7-Ethyl-17-n-propoxycarbonyloxycamptothecin-21-(2-dimethylamino) ethylamide (compound 20)

Except using n-propyl chlorocarbonate, the reaction and the after treatment were carried out according to the method of example 18 to give 241 mg (100%) of the title compound as pale yellow crystals.
mp 87-91°C (decomp)
IR (KBr) 3365, 1730, 1650, 1595, 1510, 1455, 1260 cm⁻¹
¹H-NMR (400 MHz, CDCl₃) δ 0.94 (t, J=7.3 Hz, 3H, CH₂CH₂CH₃), 1.07 (t, J=7.3 Hz, 3H, 18-CH₃), 1.35 (t, J=7.7 Hz, 3H, 7-CH₂CH₃), 1.63-1.72 (m, 2H, OCH₂CH₂CH₃), 2.17-2.50 (m, 4H, CH₂N(CH₃)₂ and 19-CH₂), 2.20 (s, 6H, N(CH₃)₂), 3.06-3.21 (m, 2H, 7-CH₂CH₃), 3.31-3.48 (m, 2H, CONHCH₂), 4.11 (t, J=6.8 Hz, 2H, OCH₂), 5.09 (s, 2H, 5-CH₂), 5.59 (s, 2H, 17-OCH₂), 7.33-7.36 (m, 1H, NH), 7.56 (s, 1H, 14-H), 7.56-7.60 (m, 1H, 10-H), 7.72-7.76 (m, 1H, 11-H), 7.99 (d, J=7.8 Hz, 1H, 9-H), 8.13 (d, J=7.8Hz, 1H, 12-H) SI-MS m/e 551(M⁺+1)

### Example 21: 17-n-Butoxycarbonyloxy-7-ethyl-camptothecin-21-(2-dimethylamino)ethylamide (compound 21)

Except using n-butyl chlorocarbonate, the reaction and the after treatment were carried out according to the method of example 18 to give 261 mg (100%) of the title compound as pale yellow crystals.
mp 83-86°C (decomp)
IR (KBr) 3370, 1740, 1645, 1595, 1510, 1455, 1255 cm⁻¹
¹H-NMR (400 MHz, CDCl₃) δ 0.91 (t, J=7.3 Hz, 3H, OCH₂CH₂CH₂CH₃), 1.06 (t, J=7.2 Hz, 3H, 18-CH₃), 1.36 (t, J=7.7 Hz, 3H, 7-CH₂CH₃), 1.37-1.43 (m, 2H, OCH₂CH₂CH₂CH₃), 1.59-1.67 (m, 2H, OCH₂CH₂CH₂CH₃), 2.21-2.51 (m, 4H, CH₂N(CH₃)₂ and 19-CH₂), 2.26 (s, 6H, N(CH₃)₂), 3.07-3.17 (m, 2H, 7-CH₂CH₃), 3.32-3.51 (m, 2H, CONHCH₂), 4.15 (t, J=6.8 Hz, 2H, OCH₂), 5.10 (s, 2H, 5-CH₂), 5.60 (s, 2H, 17-OCH₂), 7.35-7.39 (m, 1H, NH), 7.57 (s, 1H, 14-H), 7.57-7.61 (m, 1H, 10-H), 7.73-7.77 (m, 1H, 11-H), 7.99 (d, J=7.8 Hz, 1H, 9-H), 8.14 (d, J=7.6 Hz, 1H, 12-H)
SI-MS m/e 565(M⁺+1)

### Example 22: 7-Ethyl-10-methyl-17-methoxycarbonyloxycamptothecin-21-(2-dimethylamino)ethylamide (compound 22)

Except using 7-ethyl-17-hydroxy-10-methylcamptothecin-21-(2-dimethylamino)ethylamide and methyl chlorocarbonate, the reaction and the after treatment were carried out according to the method of example 18 to give 272 mg (100%) of the title compound as pale yellow crystals.
mp 150-153°C(decomp)
IR (KBr) 3365, 1745, 1650, 1600, 1510, 1440, 1265 cm⁻¹
¹H-NMR (400 MHz, CDCl₃) δ 1.06 (t, J=7.3 Hz, 3H, 18-CH₃), 1.35 (t, J=7.7 Hz, 3H, 7-CH₂CH₃), 2.28-2.50 (m, 4H, CH₂N(CH₃)₂ and 19-CH₂), 2.25 (s, *6H,* N(CH₃)₂), 2.58 (s, 3H, ArCH₃), 3.03-3.13 (m, 2H, 7-CH₂CH₃), 3.31-3.51 (m, 2H, CONHCH₂), 3.78 (s, 3H, OCH₃), 5.09 (s, 2H, 5-CH₂), 5.60 (s, 2H, 17-OCH₂), 7.32 (brs, 1H, NH), 7.52 (s, 1H, 14-H), 7.57 (dd, J=1.7, 8.8 Hz, 1H, 11-H), 7.72 (s, 1H, 9-H), 8.02 (d, J=8.8 Hz, 1H, 12-H)
SI-MS m/e 537(M⁺+1)

### Example 23: 7-Ethyl-11-fluoro-17-methoxycarbonyloxycamptothecin-21-(2-dimethylamino)ethylamide (compound 23)

Except using 7-ethyl-11-fluoro-17-hydroxy-10-methylcamptothecin-21-(2-dimethylamino)ethylamide and methyl chlorocarbonate, the reaction and the after treatment were carried out according to the method of example 18 to give 74 mg (17%) of the title compound as pale yellow crystals.
mp 150-159°C (decomp)
IR (KBr) 3360, 1735, 1650, 1600, 1510, 1450, 1265 cm⁻¹
¹H-NMR (400 MHz, CDCl₃) δ 1.07 (t, J=7.3 Hz, 3H, 18-CH₃), 1.37 (t, J=7.7 Hz, 3H, 7-CH₂CH₃), 2.17-2.32 (m, 2H, CH₂N(CH₃)₂), 2.41-2.48 (m, 2H, 19-CH₂), 2.20 (s, 6H, N(CH₃)₂), 3.10-3.20 (m, 2H, 7-CH₂CH₃), 3.30-3.48 (m, 2H, CONHCH₂), 3.79 (s, 3H, OCH₃), 5.13 (s, 2H, 5-CH₂), 5.58 (d, J=11.2 Hz, 1H, 17-OCH₂), 5.62 (d, J=11.2 Hz, 1H, 17-OCH₂), 7.26-7.29 (m, 1H, 10-H), 7.37-7.41 (m, 1H, NH),7.56 (s, 1H, 14-H), 7.75-7.78 (m, 1H, 12-H), 8.01-8.05 (m, 1H, 9-H)
SI-MS m/e 541(M⁺+1)

### Example 24: Preparation of a hydrochloride

100-200 mg of a corresponding camptothecin-21-(2-dimethylamino) ethylamide derivative were added 1,05 eq. of an aqueous 0.1 N HCl solution. If necessary, further purified water was added for dissolution. The solution was filtered using MILLEX-GV (0.22 mm Filter Unit), and the filtrate was lyophilized to give the desired hydrochloride.

The solubility of each hydrochloride prepared in purified water is shown in Table 1.

**Table 1:**

| **Solubility of each hydrochloride in purified water** | | | |
|---|---|---|---|
| Compound No. | Solubility(mg/ml) | Compound No. | Solubility(mg/ml) |
| Compound 1 | ≥50 | Compound 11 | ca. 5 |
| Compound 2 | ca.14 | Compound 14 | ca. 50 |
| Compound 3 | ca.11 | Compound 15 | ca. 53 |
| Compound 4 | ca.50 | Compound 16 | ≥33 |
| Compound 5 | ca.10 | Compound 18 | ≥50 |
| Compound 6 | ca.50 | Compound 19 | ≥52 |
| Compound 7 | ca.15 | Compound 20 | ≥52 |
| Compound 8 | ca.11 | Compound 21 | ≥54 |
| Compound 9 | ca.28 | | |
| Compound 10 | ca. 7 | | |

### Example 25: Anti-tumor activity

### Experimental method

5x10⁵ cells of mouse leukemia L1210 were transplanted intraperitoneally to a group of six female CDF₁ mice (7 weeks old, body weight 17-19 g). A test substance was administered intraperitoneally on day 1, 5 and 9, and its life prolonging effect was observed. In case of administration of a test substance as acid addition salt, it was dissolved in purified water. The total administration amount was 1.56 -400 mg/kg. The anti-tumor activity was expressed by the value (T/C%) in which the ratio of the mean survival days of a drug administered group (T) to the mean survival days of a not administered group (C) is multiplied by 100. In case of a life prolongation equal to or more than 125% the drug is considered to be effective. A therapeutic index was calculated by examining the least effective dose and the maximum tolerance dose.

### Experimental results

For the substances obtained in the above-mentioned examples the results of the anti-tumor activity test are shown in Tables 2-6 infra. As demonstrated in each Table, the new camptothecin derivatives according to the present invention show excellent activity compared with 7-ethylcamptothecin sodium salt as a reference compound.

**Table 2:**

| Anti-tumor activity | | | | | | | |
|---|---|---|---|---|---|---|---|
| Compound No. | Total dose (mg/kg) | | | | | | |
| | 3.13 | 6.25 | 12.5 | 25 | 50 | 100 | 200 |
| Compound 1 | 97 | 97 | 95 | 97 | 97 | 139 | 145 |
| Compound 3 | 100 | 100 | 139 | 142 | 147 | 147 | 147 |
| Reference Compound | 129 | 142 | 153 | 211 | 266 | 339(2/6) | 303(1/6) |

**Table 3:**

| Anti-tumor activity | | | | | | | |
|---|---|---|---|---|---|---|---|
| Compound No. | Total dose (mg/kg) | | | | | | |
| | 3.13 | 6.25 | 12.5 | 25 | 50 | 100 | 200 |
| Compound 5 | 88 | 98 | 120 | 137 | 151 | 168 | 176 |
| Compound 7 | 95 | 129 | 139 | 137 | 178 | 227 | 541(4/6) |
| Compound 8 | 93 | 122 | 129 | 134 | 154 | 176 | 256 |
| Compound 9 | 112 | 117 | 137 | 134 | 173 | 239 | 318 |
| Reference Compound | 134 | 139 | 141 | 200 | 222 | 410(1/6) | 244 |

**Table 4:**

| Anti-tumor activity | | | | | | | |
|---|---|---|---|---|---|---|---|
| Compound No. | Total dose (mg/kg) | | | | | | |
| | 3.13 | 6.25 | 12.5 | 25 | 50 | 100 | 200 |
| Compound 4 | 121 | 123 | 121 | 123 | 149 | 205 | 323(1/6) |
| Compound 10 | 115 | 118 | 123 | 136 | 174 | 221 | 310 |
| Compound 14 | 118 | 121 | 126 | 136 | 149 | 218 | 523(4/6) |
| Reference Compound | 126 | 146 | 151 | 215 | 249 | 513(4/6) | 362(1/6) |

**Table 5:**

| Anti-tumor activity | | | | | | | |
|---|---|---|---|---|---|---|---|
| Compound No. | Total dose (mg/kg) | | | | | | |
| | 3.13 | 6.25 | 12.5 | 25 | 50 | 100 | 200 |
| Compound 11 | 105 | 132 | 142 | 142 | 150 | 192 | 271 |
| Compound 18 | 139 | 150 | 132 | 139 | 218 | 597(5/6) | 524(4/6) |
| Reference Compound | 142 | 155 | 166 | 224 | 408(2/6) | 584(5/6) | 339(1/6) |

**Table 6:**

| Anti-tumor activity | | | | | | | |
|---|---|---|---|---|---|---|---|
| Compound No. | Total dose (mg/kg) | | | | | | |
| | 3.13 | 6.25 | 12.5 | 25 | 50 | 100 | 200 |
| Compound 19 | 126 | 129 | 126 | 129 | 190 | 388(2/6) | 29 |
| Compound 20 | 129 | 124 | 121 | 133 | 188 | 424(3/6) | 188 |
| Compound 21 | 124 | 126 | 131 | 129 | 138 | 195 | 157 |
| Reference Compound | 133 | 136 | 157 | 193 | 310(1/6) | 345(1/6) | 152 |

Stability test of new water-soluble camptothecin derivatives in water

### 1) Preparation of calibration curve:

1.05 mg of compound 1 and 1.10 mg of compound A (Note) were dissolved in 10 ml acetonitrile to give a standard solution. 20 µ1 of the standard solution was tested by HPLC (procedure condition 1). The peak area of compound 1 and compound A (Note), which is obtained from the chromatography of the standard solution is measured by an automatic integration method, and the obtained area is plotted against an amount of compound 1 and compound A (2.1 µ g and 2.2 µg) by 20 µl to obtain a calibration curve.

### (Note) Compound A: 7-Ethyl-17-acetoxycamptothecin-21-(2-dimethylamino) ethylamide hydrochloride

Calibration curve:
   Compound 1 Y=9.29×10⁻⁵X
   Compound A Y=7.45×10⁻⁵X
   [X: peak area, Y: amounts of Compound 1 and Compound A (µg)]

### [HPLC method, procedure condition 1]

Column: Inertsil ODS-3 (5-250), 40 deg.
Mobile phase: 0.01 M potassium dihydrogenphosphateacetonitrile (3:1)
Flow rate: 1.0 ml/min
Detection: UV absorptiometer (254 nm)

### 2) Stability of compound 1 and compound A in each pH:

About 1 mg (×3) of compound 1 were dissolved adding a 10 ml of phosphate buffer of pH 10.0, 7.0 or 5.4, stirred at room temperature (about 26°C) to prepare a test solution. For each 20 µ1 of the test solution at the time of dissolution, 3 hr later and 24 hr later, the tests were carried out by HPLC (procedure condition 1). The amounts of compound 1 were determined from the obtained area. The amounts of compound A in each pH were determined.

The amounts (µg/ml) of compound 1 and compound A at the time of dissolution, 3 hr later and 24 hr later in each pH are shown in Table 7.

**Table 7**

| Compound | | pH 10.0 | pH 7.0 | pH 5.4 |
|---|---|---|---|---|
| Compound 1 Compound A | at the time of dissolution | 109 | 105 | 118 |
| | 3 hr later | 106 | 105 | 118 |
| | 24 hr later | 97 | 105 | 118 |
| | at the time of dissolution | 102 | 110 | 126 |
| | 3 hr later | 79 | 110 | 126 |
| | 24 hr later | 16 | 107 | 124 |

### 3) Results:

compound 1 was stable at room temperature in the aqueous solutions of pH 7.0 and 5.4. Even after 24 hr 89% of compound 1 remained in the aqueous solution of pH 10.0. In contrast, 84% of compound A were hydrolyzed in the aqueous solution of pH 10.0 after 24 hr.

These results demonstrate that the 17-carbamide ester derivative is superior to the 17-ester derivative as regards stability in an alkaline aqueous solution.

## Claims

1. Camptothecin derivatives of the general formula (1) 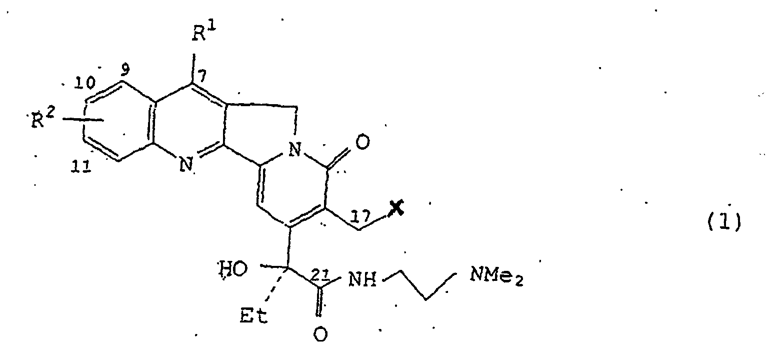 wherein R¹ represents a hydrogen atom or an alkyl group with 1-6 carbon atoms, R² represents the same or different 0-4 alkyl groups with 1-6 carbon atoms, a halogen atom, an alkoxy or hydroxyl group,
wherein X is a carbonate or a carbamate group of the formula 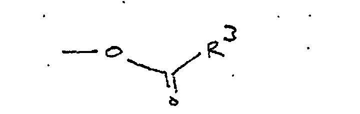 wherein R³ is selected from the group consisting of methylamino, ethylamino, propylamino, cyclohelylamino, dimethylamino, diethylamino arylamino, cyclic-amino, methoxy, ethoxy or propoxy group and salts thereof.

2. Camptothecin derivatives according to claim 1, wherein R² is a fluoro group in the 11-position of the general formula.

3. Camptothecin derivatives according to claim 1, wherein R² is a methyl group in the 10-position of the general formula.

4. Camptothecin derivatives according to claim 1, wherein R² is a fluoro group in the 11-position and a methyl group in the 10-position of the general formula.

5. Camptothecin derivatives according to claim 1, wherein R³ is a phenylamino, group.

## Patentansprüche

1. Camptothecinderivate der allgemeinen Formel (1.) 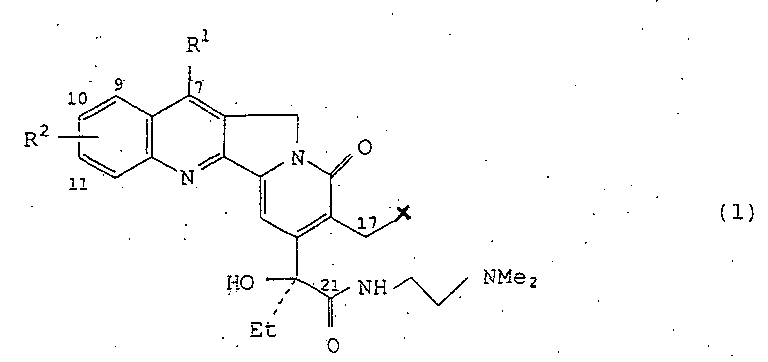 worin R¹ ein Wasserstoffatom oder eine Alkylgruppe mit 1-6 Kohlenstoffatomen darstellt, R² die gleichen oder unterschiedliche 0-4 Alkylgruppen mit 1-6 Kohlenstoffatomen, ein Halogenatom, eine Alkoxy- oder Hydroxylgruppe darstellt, worin X eine Carbonat- oder Carbamatgruppe der Formel 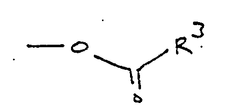 ist, worin R³ ausgewählt ist aus der Gruppe bestehend aus Methylamino-, Ethylamino-, Propylamino-, Cyclohexylamino-, Dimethylamino-, Diethylamino-, Arylamino-, cyclische Amino-, Methoxy-, Ethoxy- oder Propoxygruppen, und Salze davon.

2. Camptothecinderivate gemäß Anspruch 1, worin R² eine Fluorgruppe in der 11-Position der allgemeinen Formel ist.

3. Camptothecinderivate gemäß Anspruch 1, worin R² eine Methylgruppe in der 10-Position der allgemeinen Formel ist.

4. Camptothecinderivate gemäß Anspruch 1, worin R² eine Fluorgruppe in der 11-Position und eine Methylgruppe in der 10-Position der allgemeinen Formel ist.

5. Camptothecinderivate gemäß Anspruch 1, worin R³ eine Phenylaminogruppe ist.

## Revendications

1. Dérivés de camptothécine de formule générale (1) 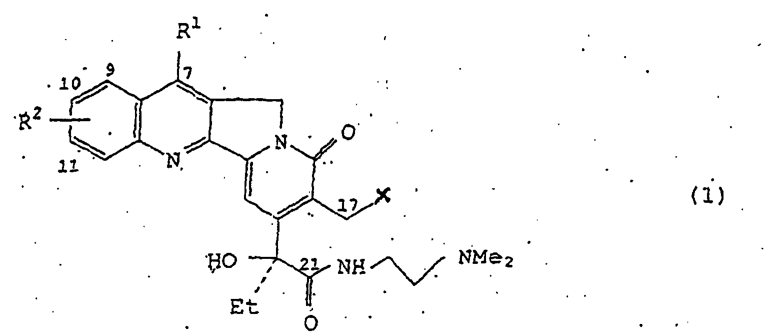 où R¹ représente un atome d'hydrogène ou un groupe alkyle ayant 1-6 atomes de carbone, R² représente 0-4 groupes alkyle identiques ou différents ayant 1-6 atomes de carbone, un atome d'halogène, un groupe alcoxy ou un groupe hydroxyle, tandis que X est un groupe carbonate ou carbamate de formule 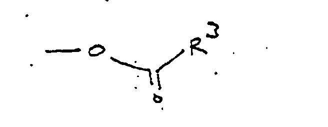 où R³ est choisi dans le groupe consistant en des groupes méthylamino, éthylamino, propylamino, cyclohexylamino, diméthylamino, diéthylamino, arylamino, amino cyclique, méthoxy, éthoxy ou propoxy, et leurs sels.

2. Dérivés de camptothécine selon la revendication 1, dans lesquels R² est un groupe fluoro dans la position 11 de la formule générale.

3. Dérivés de camptothécine selon la revendication 1, dans lesquels R² est un groupe méthyle dans la position 10 de la formule générale.

4. Dérivés de camptothécine selon la revendication 1, dans lesquels R² est un groupe fluoro dans la position 11 et un groupe méthyle dans la position 10 de la formule générale.

5. Dérivés de camptothécine selon la revendication 1, dans lesquels R³ est un groupe phénylamino.
